# FASCICULE DE BREVET EUROPEEN

(11) **EP 1 346 707 B1**
(45) Date de publication et mention de la délivrance du brevet: **14.12.2005**
(21) Numéro de dépôt: 03290154.8
(22) Date de dépôt: 22.01.2003
(51) Int. Cl.: A61F 2/30, A61F 2/46, A61L 31/14

(54) **Obturateur diaphysaire associé à un drain**
Mit einem Drainagesystem verbundener diaphysealer Verschlussstopfen
Diaphysial plug joined to a drainage system

(30) Priorité: 20.03.2002 FR 0203521
(43) Date de publication de la demande: 24.09.2003
(73) Titulaire: Centerpulse France S.A., 25461 Etupes (FR)
(72) Inventeur: Bouraly, Jean-Pierre, c/o Cabinet Ballot, 25000 Besancon (FR)
(74) Mandataire: Somnier, Jean-Louis

(56) Documents cités:
- EP-A- 0 626 155
- EP-A- 0 727 197
- WO-A-01/08569
- WO-A-93/08769
- DE-U- 9 319 132
- FR-A- 2 763 500

## Description

La présente invention concerne un obturateur ou bouchon diaphysaire utilisé dans le domaine de la prothèse de la hanche.

Lors d'une telle intervention, on sectionne le col fémoral et on procède à la préparation du fût fémoral destiné à recevoir la prothèse à l'aide d'alésoirs et de râpes.

Selon une technique opératoire connue, la prothèse est scellée par l'intermédiaire d'un ciment.

Pour cela, on prédispose dans le fût fémoral qui est creux, à une profondeur prédéterminée, un bouchon diaphysaire ou obturateur afin de permettre l'introduction du ciment sous pression, par injection à la seringue ou manuellement. L'obturateur est mis en place à une hauteur prédéterminée, en général égale à la hauteur de la tige prothétique, plus un centimètre et demi.

Différents obturateurs de ce type sont connus et sont réalisés, soit dans un matériau non résorbable, comme le polyéthylène ou une matière plastique biocompatible en général, soit dans un matériau résorbable, comme la gélatine, l'acide lactique de type vicryl ou tout copolymère résorbable.

Un inconvénient majeur dans l'utilisation de la matière plastique, pour la réalisation d'obturateurs en matière plastique, réside dans le fait que celle-ci génère lors de son vieillissement, quand la tige se descelle même partiellement, des particules qui provoquent des résorptions, c'est à dire une raréfaction de l'os par réaction des macrophages.

De plus, lors d'une éventuelle révision, il faut à la fois percer le ciment, puis essayer de récupérer l'obturateur en matière plastique, qui ne s'est donc pas résorbé dans le temps, ce qui a pour effet de rallonger le temps de cette intervention.

C'est pour ces différentes raisons que sont généralement préférés les obturateurs résorbables précités, car ils permettent d'éviter les inconvénients ci-dessus. De plus, en général, par leur élasticité, ils permettent une meilleure obturation.

Quoi qu'il en soit, dans un cas comme dans l'autre, il existe à l'heure actuelle, un inconvénient commun aux deux types d'obturateurs qui réside dans le fait qu'en cas de révision ultérieure, la zone située entre l'extrémité de la tige et l'obturateur sera encombré de ciment résiduel formant un culot gênant. Dans cette nouvelle situation, la technique de reprise peut nécessiter après l'explantation, de mettre en place une tige de reprise plus longue. Il est alors nécessaire d'éliminer le culot de ciment. Celui-ci doit être de 1,5 cm.

De plus, lors de l'injection, la pression exercera sur l'obturateur, une force ayant pour effet de le repousser, parfois jusqu'à cinq centimètres dans la cavité fémorale, ce qui provoque un culot de ciment résiduel encore plus important, pour une révision plus difficile également.

Un autre inconvénient réside dans la préparation du fût fémoral qui s'effectue généralement à l'aide d'une râpe, le saignement de l'os, s'écoulant vers le fond de la cavité fémorale, s'accumule au niveau de l'obturateur, ce qui aura pour conséquence un mauvais cimentage, le ciment lors de son introduction se mélange alors avec le sang.

Pour remédier à cela, il est connu d'utiliser un drain flexible percé à sa partie inférieure pour aspirer et évacuer les matières en présence dans la zone du fond de la cavité fémorale, telles que sang, sérum physiologique, ciment résiduel, mais le mauvais guidage du drain ne permet pas toujours d'aller réellement au fond de la cavité.

D'où la solution connue d'un obturateur comportant des moyens de liaison mécanique avec un drain permettant l'aspiration des matières précitées, simultanément à la pose de l'obturateur et à l'introduction du ciment.

Mais un tel obturateur est nécessairement en matière plastique afin de permettre la réalisation des moyens de liaison mécanique, avec bien entendu les inconvénients liés à la matière plastique, non résorbables, évoqués précédemment.

De plus, la réalisation de ces moyens nécessite des opérations d'usinage de l'obturateur, longues et coûteuses, ainsi que la mise en oeuvre de dispositifs également coûteux.

Il aurait pu paraître évident de résoudre ces problèmes en recourant aux techniques de moulage, mais il y a incompatibilité à surmouler un drain en matière plastique avec un obturateur également en matière plastique, pour des problèmes de températures et de points de fusion qui sont identiques et qui de plus auraient pour effet de rendre indissociables les deux éléments qui se trouveraient ainsi soudés et donc ne plus pouvoir retirer le drain après pose de l'obturateur et injection du ciment.

Selon une première phase de la démarche inventive, il a été estimé que ces différents problèmes pourraient être résolus d'une part en utilisant un obturateur réalisé en un matériau résorbable, avec les avantages qui en découlent par rapport à la matière plastique, et d'autre part en imaginant des moyens de liaison d'un drain en matière plastique dont la mise en place par rapport à l'obturateur s'effectuerait par l'intermédiaire de moyens de liaison qui s'affranchiraient du fait que ledit obturateur est réalisé dans un matériau qui n'est pas usinable du fait de sa très faible dureté.

La seconde phase de la démarche inventive a résidé dans le fait d'exploiter avantageusement le fait que le point de fusion d'une matière résorbable, telle que le vicryl par exemple, qui est de l'ordre de 60 - 70 °C, donc parfaitement compatible avec celui de la matière plastique du drain qui est de l'ordre de 130°C, et conséquemment sans risque de fusion de celui-ci au cours d'une opération de moulage de ce dernier.

Ainsi, l'invention comme définie dans la revendication 1 consiste en un obturateur diaphysaire destiné au scellement d'une prothèse dans le fût fémoral d'un patient, par l'intermédiaire d'un ciment, pour permettre lors de son injection la retenue de celui-ci à une profondeur prédéterminée par rapport à l'extrémité distale de la prothèse, tout en permettant l'aspiration et conséquemment l'évacuation du ciment résiduel et de toutes matières en présence dans le fond de la cavité fémorale, sang, sérum physiologique, par l'intermédiaire d'un drain assujetti à l'obturateur par des moyens de liaison, caractérisé en ce que l'obturateur est obtenu par moulage d'une matière résorbable, alors que le drain est constitué par un profilé tubulaire en matière plastique préalablement obtenu par extrusion, la liaison entre le drain en matière plastique et l'obturateur en matière résorbable étant réalisé par surmoulage du drain au cours de l'opération de moulage de l'obturateur, le point de fusion du matériau de ce dernier étant inférieur à celui de la matière plastique constituant le drain, afin de rendre compatible la liaison entre les deux éléments.

La présente invention concerne également les caractéristiques qui ressortiront au cours de la description qui va suivre.

Cette description donnée à titre d'exemple non limitatif, fera mieux comprendre comment l'invention peut être réalisée en référence aux dessins annexés sur lesquels:
La figure 1 représente une vue en coupe d'un obturateur associé à un drain selon l'invention.

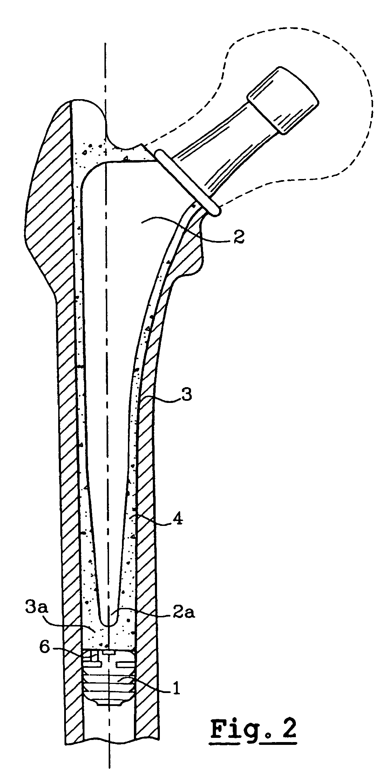

La figure 2 représente l'obturateur selon la figure 1 après mise en place dans le fût fémoral et après scellement de la tige prothétique.

L'obturateur diaphysaire 1 représenté sur les figures et désigné globalement, est destiné au scellement d'une prothèse 2 dans le fût fémoral 3 d'un patient, par l'intermédiaire d'un ciment 4.

Ceci a pour but de permettre lors de l'injection du ciment ou de son introduction manuelle dans le fût fémoral 3 de le retenir à une profondeur prédéterminée par rapport à l'extrémité distale 2a de la prothèse 2.

De manière connue, l'obturateur 1 est assujetti par des moyens de liaison à un drain 5 pour permettre l'aspiration et conséquemment l'évacuation du sang et de toutes autres matières en présence dans le fond 3a de la cavité fémorale 3.

Selon l'invention, permettant de résoudre les divers problèmes évoqués ci-dessus, l'obturateur 1 est obtenu par moulage d'une manière résorbable tels que la gélatine, le vicryl, ou tous copolymères résorbables alors que le drain 5 est constitué par un profilé tubulaire en matière plastique préalablement obtenue par extrusion, (Polychlorure de vinyl ou autre).

Il n'est pas connu à ce jour des obturateurs en matériau résorbable associés à un drain en matière plastique.

Selon une autre caractéristique essentielle de l'invention, la liaison entre le drain 5 en matière plastique et l'obturateur 1 en matière résorbable est réalisée par surmoulage dudit drain 5 au cours de l'opération de moulage de l'obturateur 1.

Le matériau résorbable constituant l'obturateur 1 est choisi en fonction de son point de fusion, de manière telle que celui-ci soit inférieur au point de fusion de la matière plastique constituant le drain 5, afin de rendre compatible la liaison entre les deux éléments précités.

Selon une autre caractéristique de l'invention, la différence entre les points de fusion du matériau résorbable de l'obturateur 1 et de la matière plastique constituant le drain 5, est telle à permettre une liaison entre les deux éléments lors de l'opération de surmoulage assurant une adhérence relative de l'un par rapport à l'autre pour retenir le drain 5 sur l'obturateur 1 lors de la mise en place de celui-ci et pouvoir l'extraire par traction manuelle après sa mise en place et évacuation par aspiration du ciment résiduel et de toutes matières en présence dans la cavité fémorale 3.

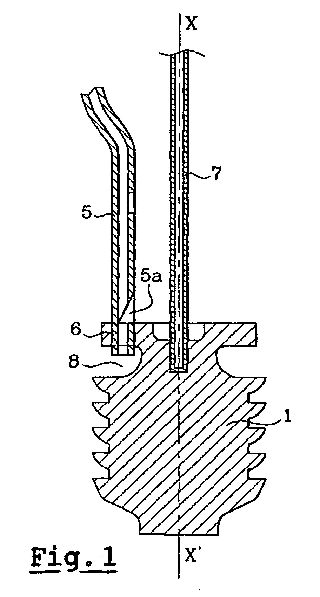

Comme le montre la figure 1, l'obturateur 1 comporte un trou 6 perpendiculaire à son axe longitudinal X, X' se formant au cours de moulage, par la présence même du drain 5 dans le moule, au cours de cette opération, d'où le surmoulage recherché.

Le drain 5 comporte un évent 5a réalisé préalablement, à sa partie inférieure et positionné au-dessus du plan supérieur de l'obturateur 1. Le drain 5 débouche dans un espace annulaire 8 réalisé à la partie supérieure de l'obturateur 1 après avoir traversé le trou 6.

De cette manière, l'obturateur 1 associé au drain 5 par surmoulage peut être mis en place, lors d'une intervention, dans la cavité fémorale par l'intermédiaire de l'ancillaire 7 qui sera ultérieurement retirée, ainsi que le drain 5 et ceci par simple traction manuelle sur celui-ci après que l'opération de drainage ait été effectuée.

## Revendications

1. Obturateur diaphysaire destiné au scellement d'une prothèse (2) dans le fût fémoral (3) d'un patient, par l'intermédiaire d'un ciment (4), pour permettre lors de son injection la retenue de celui-ci a une profondeur prédéterminée par rapport à l'extrémité distale (2a) de la prothèse (2), tout en permettant l'aspiration et conséquemment l'évacuation du sang et de toutes matières en présence dans le fond (3a) de la cavité fémorale (3), par l'intermédiaire d'un drain (5) assujetti à l'obturateur (1) par des moyens de liaison, **caractérisé en ce que** l'obturateur (1) est obtenu par moulage d'une matière résorbable, alors que le drain (5) est constitué par un profilé tubulaire en matière plastique préalablement obtenu par extrusion, la liaison entre le drain (5) en matière plastique et l'obturateur (1) en matière résorbable étant réalisé par surmoulage du drain (5) au cours de l'opération de moulage de l'obturateur (1), le point de fusion de matériau de ce dernier étant inférieur à celui de la matière plastique constituant le drain (5), afin de rendre compatible la liaison entre les deux éléments (1, 5)

2. Obturateur diaphysaire selon la revendication 1, **caractérisé en ce que** la différence entre les points de fusion du matériau résorbable de l'obturateur (1) et de la matière plastique constituant le drain (5) est telle à permettre une liaison entre les deux éléments lors de l'opération de surmoulage assurant une adhérence relative de l'un par rapport à l'autre pour retenir le drain (5) sur l'obturateur (1) lors de la mise en place de celui-ci et pouvoir l'extraire par traction manuelle après sa mise en place et évacuation par aspiration du sang et de toutes matières en présence dans la cavité fémorale (3).

## Patentansprüche

1. Diaphysealer Verschlußstopfen zum Einzementieren einer Prothese (2) im femoralen Schaft (3) eines Patienten mittels eines Zements (4), um bei seinem Injizieren das Halten dieses auf einer vorbestimmten Tiefe im Verhältnis zum distalen Ende (2a) der Prothese (2) zu ermöglichen, wobei gleichzeitig das Absaugen und folglich der Abtransport des Blutes und der gesamten sich im hinteren Teil (3a) der femoralen Aushöhlung (3) befindenden Materialien mittels eines am Verschlußstopfen (1) mittels Verbindungsmitteln befestigten Drains (5) ermöglicht ist, **dadurch gekennzeichnet, daß** der Verschlußstopfen (1) durch Formen eines resorbierbaren Materials erhalten wird, während der Drain (5) durch ein tubuläres Profil aus einem zuvor mittels Extrusion erhaltenen Kunststoffmaterial gebildet ist und die Verbindung zwischen dem Drain (5) aus Kunststoffmaterial und dem Verschlußstopfen (1) aus resorbierbarem Material durch Überformen des Drains (5) im Verlauf des Formungsschritts des Verschlußstopfens (1) hergestellt wird, wobei die Schmelztemperatur des Materials des letzteren niedriger ist als diejenige des den Drain (5) bildenden Kunststoffmaterials, um die Verbindung zwischen den beiden Elementen (1, 5) kompatibel zu machen.

2. Diaphysealer Verschlußstopfen gemäß Anspruch 1, **dadurch gekennzeichnet, daß** die Differenz zwischen den Schmelztemperaturen des resorbierbaren Materials des Verschlußstopfens (1) und des den Drain (5) bildenden Kunststoffmaterials so ist, daß eine Verbindung zwischen den beiden Elementen beim Überformungsschritt möglich wird, der ein relatives Anhaften des einen im Verhältnis zum anderen sicherstellt, um den Drain (5) auf dem Verschlußstopfen (1) beim Positionieren desselben zu halten und um diesen durch manuelles Ziehen nach seiner Positionierung und nach dem Abtransport durch Absaugen des Bluts und aller sich in der femoralen Aushöhlung (3) befindenden Materialien herausziehen zu können.

## Claims

1. Diaphysial plug (1) designed to seal a prosthesis (2) in a patient's femoral canal (3) by means of a cement (4) such that, when the latter is injected, it is retained at a predetermined depth in relation to the distal end (2a) of the prosthesis (2), while at the same time permitting aspiration and, consequently, evacuation of the blood and all the substances present at the bottom (3a) of the femoral cavity (3), by means of a drain (5) connected to the plug (1) by connecting means, **characterized in that** the plug (1) is obtained by moulding of an absorbable material, while the drain (5) is formed as a tubular profile of plastic obtained beforehand by extrusion, the connection between the drain (5) made of plastic material and the plug (1) made of absorbable material being produced by overmoulding of the drain (5) during the operation of moulding the plug (1), the melting point of the material of the latter being lower than that of the plastic material constituting the drain (5), in order to make the connection between the two elements (1, 5) compatible.

2. Diaphysial plug according to Claim 1, **characterized in that** the difference between the melting points of the absorbable material of the plug (1) and of the plastic material of the drain (5) is such as to permit a connection between the two elements during the overmoulding operation, ensuring adhesion of the two parts relative to one another such as to retain the drain (5) on the plug (1) during fitting of the latter and such as to allow manual extraction thereof after it has been fitted and also evacuation, by aspiration, of the blood and all the materials present in the femoral cavity (3).
